# EUROPEAN PATENT APPLICATION

(11) **EP 2 657 221 A1**
(43) Date of publication of application: **30.10.2013**
(21) Application number: 13167359.2
(22) Date of filing: 20.11.2009
(51) Int. Cl.: C07C 209/28, C07C 249/02, C07C 211/42, C07C 251/20, A61K 31/135

(54) **Preparation of rasagiline and salts thereof**

(30) Priority: 20.11.2008 IN CH28822008; 12.01.2009 US 143996 P; 13.07.2009 IN CH16502009
(62) Divisional of application: 09828275.9
(71) Applicant: Dr. Reddy's Laboratories Ltd., Hyderabad 500 016, Andhra Pradesh (IN); Dr. Reddy's Laboratories, Inc., Bridgewater, NJ 08807 (US)
(72) Inventor: Cherukupally, Praveen, 500 039 Andhra Pradesh (IN); Vajrala, Venkata, Reddy, 500 050 Andhra Pradesh (IN); Adla, Vijaya, Kumar, 506 303 Andhra Pradesh (IN); Rangineni, Srinivasulu, 509 102 Andhra Pradesh (IN); Kanniah, Sundaralakshmi, 632 001 Tamilnadu (IN)
(74) Representative: Bates, Philip Ian

(57) **Abstract**

Aspects of the present invention relate to rasagiline, proceeses for the preparation of rasagiline, and pharmaceutically acceptable salts thereof.

## Description

### INTRODUCTION

Aspects of the present application relate to rasagiline, processes for the preparation of rasagiline, and pharmaceutically acceptable salts thereof.

The drug compound having the adopted name "rasagiline mesylate" has a chemical name (1R)-N-prop-2-ynyl-2,3-dihydro-1H-inden-1-amine methanesulfonate, and can be represented by structural Formula I.

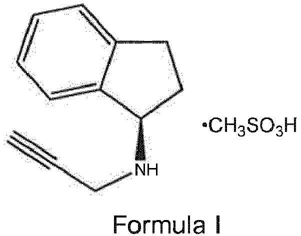

Rasagiline mesylate is prescribed for the treatment of idiopathic Parkinson's disease.

U.S. Patent Nos. 3,253,037, 5,457,133, and 5,532,415 pertain to racemic rasagiline, its enantiomer and a mesylate salt.

Chinese Patent Application No. 1990455 A describes a process for the preparation of rasagiline via reductive amination in the presence of hydrogen gas or borohydride. The process of the Chinese patent application has serious problems, such as a long reaction time (24 hours), and low yields and purity.

U.S. Patent No. 7,375,249 discloses a process for preparation of rasagiline wherein 1-indanone is reduced under chiral reducing condition to enantiomerically pure 1-indanol, followed by activation of the hydroxyl group and its subsequent reaction with propargyl amine. U.S. Patent No. 7,491,847 discloses a process for isolation of rasagiline from a reaction mixture comprising solvent, primary-aminoindan and tertiary-aminoindan.

International Application Publication No. WO 2008/076348 A1 discloses crystalline rasagiline free base, a process for its preparation and a pharmaceutical composition comprising crystalline rasagiline free base.

Chinese Patent Application No. 101486655 A and International Application Publication No. WO 2009/118657 A2 disclose polymorphs of rasagiline mesylate.

U.S. Patent Application Publication No. 2006/0188581 is directed to a mixture of particles of pharmaceutically acceptable salts of Rasagiline, wherein more than 90% of the total amount by volume of particles of Rasagiline salt have a size less than 250 µm. The application emphasizes the concern of maintaining uniformity of content during formulation of low dosage strength drugs like Rasagiline mesylate. It also highlights that the large, irregularly shaped particles arising from salt crystallization can easily decrease content uniformity in formulated products.

International Application Publication No. WO 2009/122301 discloses rasagiline mesylate having 90 volume-percent of the particles (D₉₀) with sizes about 600 µm to about 1500 µm, and a process for its preparation.

The solid state physical properties of an active pharmaceutical ingredient (API), such as rasagiline mesylate, can be very important in formulating pharmaceutical products, having profound effects on the ease of formulating and reproducibility of formulations. Particle sizes, for example, may affect the flowability and mixability of a drug substance. Additionally, pharmaceutical stability is believed to depend on simultaneous influence of various factors, of which some important factors include the sizes of crystals, shapes of crystals, water content, residual solvents, and impurities.

Although several processes have been reported for the preparation of rasagiline and its salts, they suffer from one or more drawbacks such as low yields, long reaction duration, and use of expensive chiral reducing agents. Hence, there remains a need for simple, cost effective and industrially viable processes for the production of rasagiline and its pharmaceutically acceptable salts.

### SUMMARY

Aspects of the present invention provide processes for the preparation of rasagiline and its pharmaceutically acceptable salts, substantially free from chiral and process related impurities, embodiments comprising at least one of the steps:
(a) reacting 1-indanone of Formula II with propargylamine or its salt, in the presence of a suitable solvent, to afford N-(2-propynyl)-indanyl-imine ("imine intermediate") of Formula III; and 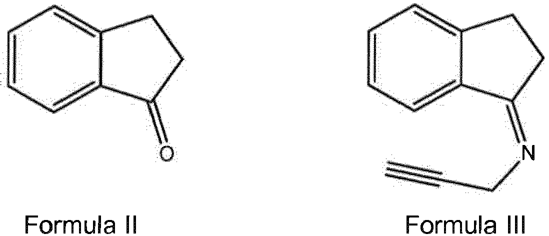
(b) reducing the imine intermediate with a suitable reducing agent, under suitable reaction conditions, to afford racemic rasagiline of Formula IV.

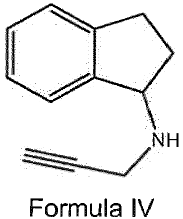

An aspect of the present invention includes processes for resolution of racemic or an enantiomerically enriched rasagiline to obtain a desired enantiomer, embodiments comprising at least one of the steps:
(a) resolving racemic or an enantiomerically enriched rasagiline, in the presence of a suitable chiral resolving agent, to afford a diasteromeric salt;
(b) optionally, obtaining a free base of an enantiomer from its corresponding diastereomeric salt obtained in (a); and
(c) reacting the free base obtained in (b), or diastereomeric salt of (a), with a pharmaceutically acceptable acid in a suitable solvent, to afford a pharmaceutically acceptable acid addition salt of rasagiline.

In an aspect, the present invention provides particle size distributions of rasagiline mesylate of Formula I, wherein more than about 90% of the particles have sizes less than or equal to about 6 µm.

In an aspect, the present invention provides processes for the preparation of uniform particle size distributions of rasagiline mesylate of Formula I, embodiments comprising:
a) providing a solution of rasagiline mesylate in isopropyl alcohol;
b) combining the solution of a) with an anti-solvent;
c) recovering solid rasagiline mesylate from (b); and
d) reducing particle sizes of rasagiline mesylate to obtain a D₉₀ value less than about 6 µm.

In an aspect, the present invention provides processes for the preparation of uniform particle size distributions of rasagiline mesylate of Formula I, embodiments comprising:
a) slurrying rasagiline mesylate in methyl t-butyl ether;
b) recovering solid rasagiline mesylate from a); and
c) reducing particle sizes of rasagiline mesylate to obtain a D₉₀ value less than about 6 µm.

### DETAILED DESCRIPTION

The terms such as "about," "generally," "substantially," and the like are to be construed as modifying a term or value such that it is not an absolute. When a molecule or other material is identified herein as "substantially pure," it generally means, unless specified otherwise, that the material is at least about 95% pure, as determined by methods that are conventional in the art such as high performance liquid chromatography (HPLC) or spectroscopic methods. "Pure" generally means, unless specified otherwise, that the material is at least about 99% pure, as determined by such conventional methods. In general, this refers to purity with regard to unwanted residual solvents, reaction by-products, impurities, and unreacted starting materials.

In the case of stereoisomers, "substantially pure" refers to a compound comprising less than about 5% of other isomers of the same compound, or less than about 3%, or less than about 2%, or less than about 0.5%. Similarly, "enantiomerically enriched" refers to a mixture that may be racemic or may have a weight ratio greater than about 4:1 to about 1:4 of rasagiline to another isomer that is an S-isomer analog.

Percentage values are used herein to denote percent by weight, unless the context indicates otherwise.

Aspects of the present invention include processes for the preparation of rasagiline and its pharmaceutically acceptable salts, substantially free from chiral and process related impurities, embodiments comprising at least one of the steps:
(a) reacting 1-indanone of Formula II with propargylamine or its salt in the presence of a suitable solvent, to afford N-(2-propynyl)-indanylimine ("imine intermediate") of Formula III; and 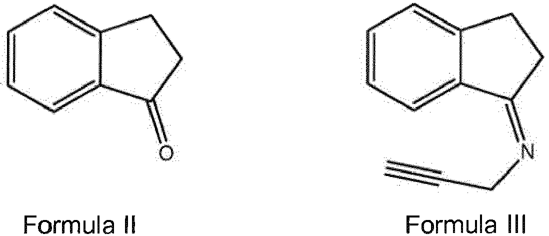
(b) reducing the imine intermediate with a suitable reducing agent, under suitable reaction conditions, to afford racemic rasagiline of Formula IV.

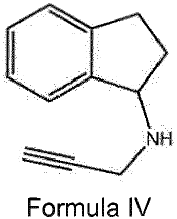

Step (a) involves reacting 1-indanone of Formula II with propargylamine or its salt in the presence of a suitable solvent, to afford the imine intermediate of Formula III.

Suitable solvents for use in step (a) include, but are not limited to: alcohols, such as, for example, methanol, ethanol, isopropanol, n-propanol, and the like; hydrocarbons, such as, for example, toluene, xylene, n-heptane, cyclohexane, and the like; mixtures thereof; and combinations thereof with water in various proportions.

Step (a) may be accomplished at temperatures about 10-90°C, such as, for example, about 35-40°C.

Propargylamine used in (a) may be in the form of the free base or an acid addition salt. For example, step (a) may employ propagylamine hydrochloride.

Optionally, the imine intermediate of Formula III may be isolated in the form of an acid addition salt and further purified by recrystallization, to achieve the desired purity.

Step (b) involves reduction of the imine intermediate Formula III with a suitable reducing agent under suitable reaction conditions, to afford racemic rasagiline of Formula IV.

Suitable reducing agents include, but are not limited to: metal catalysts, such as, for example, Raney nickel, palladium on carbon, and platinum dioxide; lithium aluminium hydride; sodium borohydride (NaBH₄); sodium cyanoborohydride (NaBH₄CN); NaBH₄ in acidic conditions; and sodium bis(2-methoxyethoxy)aluminum hydride (e.g., VITRIDE®).

Suitable solvents that can be used in this step include, but are not limited to: alcohols, such as, for example, C₁-C₄ alcohols; halogenated solvents, such as, for example, C₁-C₆ straight chain branched or aromatic halogenated hydrocarbons, *e.g.,* dichloromethane, ethylene dichloride, chloroform, carbon tetrachloride, chlorobenzene, dichlorobenzene, and the like; hydrocarbons, such as, for example, toluene, xylene, cyclohexane, and the like; nitriles, such as, for example, acetonitrile, propionitrile, and the like; aprotic solvents, such as, for example, dimethylsulphoxide (DMSO), N,N-dimethylformamide (DMF), N,N-dimethylacetamide (DMAC), N-methylpyrrolidone (NMP), and the like; mixtures thereof; and combinations thereof with water in various proportions.

Step (b) may be conducted at temperatures about 10-75°C, or about 20-40°C.

Optionally, both of steps (a) and (b) can be carried out in a single vessel, by generation of the imine and its subsequent *in situ* reduction with a suitable reducing agent.

Alternatively, both of steps (a) and (b) can be carried out stereoselectively to obtain an enantiomerically enriched or substantially enantiomerically pure R-propargylaminoindan.

The product compound can be separated from the reaction mixture by adjusting the pH to values above about 8 with a base, followed by extraction of the compound into a suitable water immiscible organic solvent.

Suitable solvents that can be used for extraction of the compound include, but are not limited to: C₃-C₆ ketones, such as, for example, ethyl methyl ketone and diethyl ketone; halogenated hydrocarbons, such as, for example, dichloromethane, ethylene dichloride, chloroform, and carbon tetrachloride; esters, such as, for example, ethyl acetate, isopropyl acetate, t-butyl acetate; mixtures thereof; and combinations thereof with water in various proportions.

The racemic rasagiline free base can optionally be subjected to diastereomeric salt formation in the same flask by reaction of racemic rasagiline free base with a suitable chiral resolving agent, to form its diastereomeric salt.

Optionally, the racemic rasagiline may be converted to an acid addition salt and further purified by recrystallization to achieve the desired purity.

Embodiments of the present invention include processes for resolution of racemic or enantiomerically enriched rasagiline to obtain a desired enantiomer of rasagiline, comprising at least one of the steps:
(a) resolving racemic or enantiomerically enriched rasagiline in the presence of a suitable chiral resolving agent to afford a diasteromeric salt;
(b) optionally, obtaining the free base of an enantiomer from its corresponding diastereomeric salt obtained in step (a); and
(c) reacting the free base obtained in step (b) or diastereomeric salt of step (a) with a pharmaceutically acceptable acid in a suitable organic solvent to afford a pharmaceutically acceptable acid addition salt of rasagiline.

Resolution step (a) involves resolving racemic or enantiomerically enriched rasagiline in the presence of a suitable chiral resolving agent to afford a diastereomeric salt.

Suitable chiral resolving agents include, but are not limited to: a tartaric acid, such as L-(+)-tartaric acid and (-)-di-*p*-toluoyltartaric acid ("DPTTA"); a mandelic acid, such as D-(-)-mandelic acid; a camphorsulphonic acid, such as (-)-camphor-10-sulphonic acid; (S)-6-methoxy-α-methyl-2-naphthaleneacetic acid (S-naproxen); (S)-(+)-ibuprofen (dexibuprofen); and the like.

Suitable organic solvents for resolution step (a) include, but are not limited to: alcohols, such as, for example, methanol, ethanol, isopropanol, n-butanol, and the like; ketones, such as, for example, acetone, ethyl methyl ketone, methyl isobutyl ketone, and the like; esters, such as, for example, ethyl acetate, n-propyl acetate, n-butyl acetate, t-butyl acetate, and the like; nitriles, such as, for example, acetonitrile, propionitrile, and the like; halogenated hydrocarbons, such as, for example, dichloromethane, ethylene dichloride, chloroform, and the like; aprotic solvents, such as, for example, N,N-dimethylformamide (DMF), dimethylsulfoxide (DMSO), N,N-dimethylacetamide (DMAC), and the like; mixtures thereof; and combinations thereof with water in various proportions.

The chiral resolving agent may be combined with the solution of racemic rasagiline at temperatures about 20-120°C, or about 60-70°C.

After the addition of the chiral resolving agent, typically, for solid product formation to occur, the reaction mass may be maintained at temperatures lower than the reaction temperature, such as, for example, below about 25°C, for periods of time as desired for a more complete formation of the product. A person skilled in the art can readily determine the exact cooling temperatures and times required for complete formation.

The diastereomeric salt of rasagiline may be separated from the final mixture by any techniques, including decantation, filtration by gravity or suction, centrifugation, and the like, optionally drying the solid using conventional means of drying.

The diastereomeric salt of rasagiline obtained in resolution step (a) typically comprises less than about 10%, or less than about 5%, or less than about 1%, or less than about 0.05%, of the isomeric impurity.

Resolution step (b) involves optionally obtaining a free base of an enantiomer from its corresponding diastereomeric salt obtained in step (a), by treatment with a suitable base

Suitable solvents that can be used to dissolve a diasteromeric salt include, but are not limited to: alcohols, such as, for example, C₁-C₄ alcohols; C₂-C₆ ketones, such as, for example, acetone, ethyl methyl ketone, and diethyl ketone; halogenated solvents, such as, for example, C₁-C₆ straight chain branched or aromatic halogenated hydrocarbons, *e.g.,* dichloromethane, ethylene dichloride, chloroform, carbon tetrachloride, chlorobenzene, dichlorobenzene, and the like; hydrocarbons, such as, for example, toluene, xylene, cyclohexane, and the like; esters, such as, for example, ethyl acetate, isopropyl acetate, t-butyl acetate, and the like; nitriles, such as, for example, acetonitrile, propionitrile, and the like; aprotic solvents, such as, for example, DMSO, DMF, DMAC, NMP, and the like; mixtures thereof; and their combinations thereof with water in various proportions.

Suitable bases that can be employed include, but are not limited to: inorganic bases such as hydroxides, alkoxides and carbonates; organic bases such as pyridine, lutidine, triethylamine, 4-dimethylaminopyridine (DMAP), dicyclohexylamine, diisopropylethylamine, and the like. A specific example of a useful base is sodium hydroxide.

Suitable temperatures for conducting resolution step (b) are about 10-75°C, or about 20-40°C.

Step (c) involves reacting the rasagiline free base obtained in step (b) with a pharmaceutically acceptable acid, to afford a rasagiline salt.

Suitable solvents that can be used to dissolve the rasagiline free base include, but are not limited to: alcohols, such as, for example, C₁-C₄ alcohols; C₂-C₆ ketones, such as, for example, acetone, ethyl methyl ketone, and diethyl ketone; halogenated solvents, such as, for example, C₁-C₆ straight chain branched or aromatic halogenated hydrocarbons, *e.g.,* dichloromethane, ethylene dichloride, chloroform, carbon tetrachloride, chlorobenzene, dichlorobenzene, and the like; hydrocarbons, such as, for example, toluene, xylene, cyclohexane, and the like; esters, such as, for example, ethyl acetate, isopropyl acetate, t-butyl acetate, and the like; nitriles, such as, for example, acetonitrile, propionitrile, and the like; aprotic solvents, such as, for example, DMSO, DMF, DMAC, NMP, and the like; mixtures thereof; and combinations thereof with water in various proportions.

Suitable temperatures for conducting step (c) are about 10-75°C, or from about 20-40°C.

The present invention includes rasagiline of Formula I, substantially free from N,N-di-(2-propynyl)-indanylamine of Formula V.

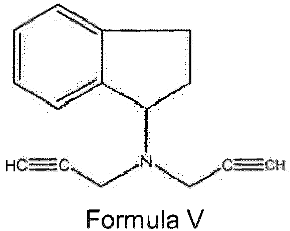

The present invention includes rasagiline of Formula I substantially free from N-allyl indanylamine of Formula VI.

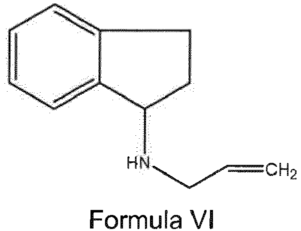

The present invention includes rasagiline of Formula I substantially free from N-propylindanylamine of Formula VII.

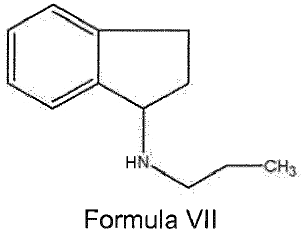

The present invention includes rasagiline or its salts substantially free from 3-propynyl-amino-1-indanone of Formula VIII or its salts. In embodiments, this impurity will be present in amounts less than about 0.05 weight percent.

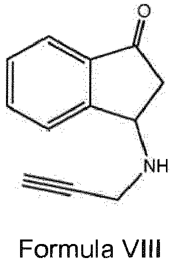

The presence of impurities in rasagiline mesylate may pose a problem for pharmaceutical product formulation, in that impurities often affect the safety and shelf life of a formulation. The present invention provides a method for ameliorating the effect of an impurity present in formulations of rasagiline Mesylate by reducing the amount of the impurities during synthesis.

The purity of product can be increased by recrystallization or slurrying of racemic Rasagiline or diastereomeric salt or any other salt of rasagiline in suitable solvents by processes known in the art. The suitable crystallization techniques include, but are not limited to: concentrating, cooling, stirring, or shaking, a solution containing the compound or by adding anti-solvent, adding seed crystals, evaporation, flash evaporation and the like. An anti-solvent as used herein refers to a solvent in which salt of rasagiline is less soluble or poorly soluble. The solvents that can be employed for crystallization include, but are not limited to lower alkanols, such as methanol, ethanol, isopropyl alcohol, esters such as ethyl acetate, n-propyl acetate, and isopropyl acetate, ethers such as 1,4-dioxane and tetrahydrofuran, nitriles such as acetonitrile, and mixtures thereof.

The pharmaceutically acceptable acid addition salts or diastereomeric salts have potential to serve as intermediates in the purification of free base or preparation of other, for example, pharmaceutically acceptable acid addition salts.

The rasagiline salt is substantially free from impurities. Typically, the rasagiline salt is of high purity, such as at least about 99%, 99.5%, or 99.9%, by weight pure. Correspondingly, the level of impurities may be less than about 1 %, 0.5%, or 0.1 %, by weight, as determined using high performance liquid chromatography (HPLC).

Rasagiline and its impurities can be analyzed by HPLC using a ZORBAX Eclipse-XDB C18 150x4.6 mm, 5 µm or equivalent column, with the following parameters:
Column temperature: 30±2°C.
Detector: UV detector.
Injection volume: 10 µL.
Flow rate: 1.0 mL/minute.
Buffer: Dissolve 1.36 grams of potassium dihydrogen phosphate and 2.44 grams of decane-1-sulfonic acid sodium salt in 1 L of water and add 1 mL of triethylamine. Adjust the pH to 2.0 with 10% v/v phosphoric acid.
Eluent A: degassed mixture of buffer, acetonitrile and methanol in a volume ratio of 80:19:1
Eluent B: degassed mixture of buffer and acetonitrile in a volume ratio of 20:80.

Gradient elution program (values in volume %):

| **Minutes** | **Eluent A** | **Eluent B** |
|---|---|---|
| 0.1 | 95 | 5 |
| 10 | 95 | 5 |
| 25 | 80 | 20 |
| 30 | 80 | 20 |
| 55 | 45 | 55 |
| 55.1 | 95 | 5 |
| 68 | 95 | 5 |

Representative relative retention times for some impurities (rasagiline = 1) are as follows:

| **Impurity** | **RRT** |
|---|---|
| 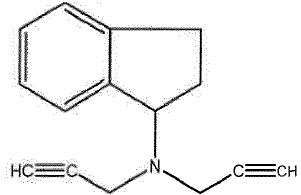 | ∼2.93 |
| 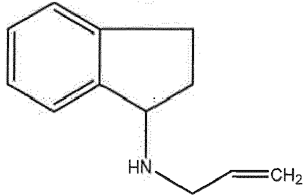 | ∼1.05 |
| 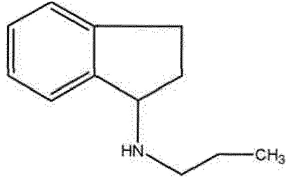 | ∼1.23 |
| 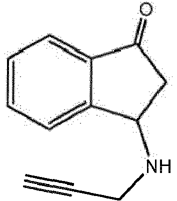 | ∼0.60 |

In an aspect, the present invention provides rasagiline mesylate of Formula I having a particle size distribution, wherein more than about 90% of the total particles have sizes less than 6 µm.

The D₁₀, D₅₀, and D₉₀ values are useful for indicating a particle size distribution. D₉₀ refers to 90 volume percent of the particles having a size smaller than the specified value. Likewise D₁₀ refers to 10 volume percent of the particles having a size smaller than the specified value. D₅₀ refers to 50 volume percent of the particles having a size smaller than the specified value. Methods for determining D_{10,} D₅₀ and D₉₀ include laser diffraction, such as using Malvern equipment (from Malvern Instruments Limited, Malvern, Worcestershire, United Kingdom).

In an aspect, the present invention provides processes for the preparation of rasagiline mesylate of Formula I having desired particle size distributions, which comprise the steps:
a) providing a solution of rasagiline mesylate in an aqueous organic solvent or organic solvent;
b) combining the solution of step a) with an anti-solvent;
c) recovering solid rasagiline mesylate; and
d) reducing particle sizes of rasagiline mesylate to obtain D₉₀ values less than about 6 µm.

Step a) involves providing a solution of rasagiline mesylate in a solvent, such as isopropyl alcohol.

The solution of rasagiline mesylate may be obtained by dissolving rasagiline mesylate in a solvent, such as isopropyl alcohol. Alternatively, the rasagiline mesylate solution may be obtained directly from a reaction in which rasagiline mesylate is formed.

Suitable solvents for step a) include, but are not limited to: alcohols, such as, for example, methanol, ethanol, isopropanol, n-butanol, and the like; ketones, such as, for example, acetone, ethyl methyl ketone, methyl isobutyl ketone, and the like; esters, such as, for example, ethyl acetate, n-propyl acetate, n-butyl acetate, t-butyl acetate, and the like; nitriles, such as, for example, acetonitrile, propionitrile, and the like; mixtures thereof; and combinations thereof with water in various proportions.

Temperatures for dissolution can range from about 25°C to about 100°C. The time period can be as long as required for a complete dissolution. Any other temperatures also are acceptable, as long as the stability of rasagiline mesylate is not compromised and a clear solution is obtained. Suitable temperatures can be about 50-90°C, or about 70-80°C.

The solution can optionally be filtered by passing through paper, glass fiber, or other membrane material, or a bed of a clarifying agent such as celite. Depending upon the equipment used and the concentration and temperature of the solution, the filtration apparatus may need to be heated to avoid premature crystallization.

Step b) involves combining the solution of step a) with an anti-solvent.

The rasagiline salt solution of step a) can be combined with a suitable anti-solvent. Suitable anti-solvents used in this step include, but are not limited to, ethers such as diethyl ether, dimethyl ether, diisopropyl ether, methyl t-butyl ether, tetrahydrofuran, 1,4-dioxane, and the like.

Suitable temperatures of an anti-solvent for combining with a rasagiline salt range from about -10°C to about 10°C. The anti-solvent is used in an amount about 2 to about 50 times the volume of the solution.

The mixture may be stirred for solid formation at temperatures such as, for example, below about 5°C to about 25°C, for periods of time as required for complete solid formation. The exact temperatures and time required for complete solid formation can be readily determined by a person skilled in the art.

Step c) involves recovering rasagiline mesylate from step b).

The methods by which the solid material is recovered from the final mixture, with or without cooling below the operating temperature, can be any of techniques such as decantation, filtration by gravity or suction, centrifugation, and the like. The isolated crystals may carry a small proportion of occluded mother liquor containing a higher percentage of impurities. If desired, the isolated crystals may be washed with a solvent to wash out the mother liquor.

The wet cake obtained may optionally be further dried. Drying can be suitably carried out in a tray dryer, vacuum oven, air oven, fluidized bed dryer, spin flash dryer, flash dryer and the like. The drying can be carried out at temperatures of about 30°C to about 90°C, with or without applying vacuum. The drying can be carried out for any desired times until the desired product purity is achieved.

Step d) involves reducing particle sizes of rasagiline mesylate salt of step c) to obtain D₉₀ values less than about 6 µm.

The obtained rasagiline mesylate obtained from step c) may have agglomerates or have poor content uniformity. The uniformity of the particles can be increased by reducing the particle sizes. Suitable techniques that can be used to reduce the particle sizes include grinding, milling, micronizing, pulverizing, etc. The injecting and milling pressure for micronisation varies with batch size.

Optionally, steps a) and b) can be replaced by a single step of slurrying, wherein rasagiline mesylate is mixed with a suitable liquid, such as methyl-t-butyl ether or another anti-solvent, followed by its recovery and micronization. The slurrying step facilitates obtaining desired particle sizes with a reduced tendency for agglomeration.

Rasagiline mesylate according to embodiments of the present invention has particle size distributions where: D₁₀ is less than about 1 µm, or less than about 3 µm; D₅₀ is less than about 5 µm, or less than about 3 µm; and D₉₀ is less than about 6 µm. There is no specific lower limit for any of the D values.

It has been observed that rasagiline mesylate of such small particle sizes is susceptible to agglomerate formation when exposed to the atmosphere. Thus, the exposure of rasagiline mesylate to the atmosphere may lead to deviation of the drug product from content uniformity requirements as a result of agglomeration. In order to enhance the uniformity in particle size, rasagiline mesylate obtained after micronization has been packaged under an inert atmosphere, such that the agglomerate formation is reduced.

A packaging and storage process for stabilizing the micronised material comprises placing micronised rasagiline mesylate in a sealed container under an inert atmosphere, such as a nitrogen atmosphere.

The present invention includes rasagiline mesylate formulated as: solid oral dosage forms, such as, for example, powders, granules, pellets, tablets, capsules; liquid oral dosage forms, such as, for example, syrups, suspensions, dispersions, emulsions; and injectable preparations, such as, for example, solutions, dispersions, freeze dried compositions Immediate release compositions may be conventional, dispersible, chewable, mouth dissolving, or flash melt preparations. Modified release compositions may comprise hydrophilic and/or hydrophobic release rate controlling substances to form matrix and/or reservoir systems. The compositions may be prepared using techniques such as direct blending, dry granulation, wet granulation, or by extrusion and spheronization. Compositions may be either uncoated, film coated, sugar coated, powder coated, enteric coated, or modified release coated.

Pharmaceutical compositions of rasagiline comprise one or more pharmaceutically acceptable excipients. Pharmaceutically acceptable excipients include, but are not limited to: diluents, such as, for example starches, pregelatinized starches, lactose, powdered celluloses, microcrystalline celluloses, dicalcium phosphate, tricalcium phosphate, mannitol, sorbitol, sugar, and the like; binders, such as, for example acacia, guar gum, tragacanth, gelatin, polyvinylpyrrolidones, hydroxypropyl celluloses, hydroxypropyl methylcelluloses, pregelatinized starches, and the like; disintegrants, such as, for example starches, sodium starch glycolate, pregelatinized starches, crospovidones, croscarmellose sodiums, colloidal silicon dioxides, and the like; lubricants, such as, for example stearic acid, magnesium stearate, zinc stearate, and the like; glidants, such as, for example colloidal silicon dioxides, and the like; solubility or wetting enhancers, such as, for example anionic, cationic, and neutral surfactants; complex forming agents, such as, for example various grades of cyclodextrins; release rate controlling agents, such as, for example hydroxypropyl celluloses, hydroxymethyl celluloses, hydroxypropyl methylcelluloses, ethyl celluloses, methyl celluloses, various grades of methyl methacrylates, waxes, and the like. Other pharmaceutically acceptable excipients include, but not limited to, film formers, plasticizers, colorants, flavoring agents, sweeteners, viscosity enhancers, preservatives, antioxidants, and the like.

Certain specific aspects and embodiments of the present invention will be explained in more detail with reference to the following examples, which are provided solely for purposes of illustration and are not to be construed as limiting the scope of the invention in any manner.

### EXAMPLE 1: PREPARATION OF N-(2-PROPYNYL)-INDANYLAMINE (FORMULA III).

1-Indanone (500 g) and methanol (2000 mL) are charged into a round bottom flask containing 416.3 g of propargylamine and stirred at 25-30°C until completion of the reaction. After completion, the reaction is quenched by adding water (5000 mL) and the mixture is extracted with ethyl acetate (2×1500 mL). The organic layer is washed with water (2×1000 mL) and distilled completely under vacuum at 65°C to afford a residue. To the residue, methanol (1000 mL) is added and the mixture is cooled to 5°C. 140 g of sodium borohydride is added in portions at about 0-10°C. The mass is heated to 25-35°C and stirred for about 6 hours, then the reaction is quenched by adding 5000 mL of water and the mass is extracted with ethyl acetate (2×1000 mL). The organic layer is washed with water (2×1000 mL) and distilled completely under vacuum to afford a residue.

To the residue, ethyl acetate (2000 mL) is added and cooled to about 5°C. Hydrogen chloride in isopropanol (18%, 450 mL) is added and stirred for about 30 minutes at 25-35°C for solid formation. The solid is filtered, suction dried for about 30 minutes, and dried at 60°C to afford racemic rasagiline hydrochloride salt.

The racemic rasagiline hydrochloride salt (170 g) is dissolved in water (100 mL), pH is adjusted to 11 by adding caustic lye solution (50 mL, 40% aq. sodium hydroxide), and the mass is extracted with ethyl acetate (2×850 mL). The organic layer is separated, washed with water (2×425 mL), and distilled completely under vacuum to afford 135 g of the title compound.

### EXAMPLE 2: PREPARATION OF RACEMIC RASAGILINE HYDROCHLORIDE.

N-(2-propynyl)-indanylimine hydrochloride (1.5 g) is placed into a round bottom flask containing methanol (5 mL), cooled to about 0-10°C, and stirred for about 15 minutes. Sodium borohydride (0.13 g) is added to the solution and stirred for about 25-35°C until completion of the reaction. Water (20 mL) is added to the mixture and the reaction is quenched by adding acetic acid (2 mL) and stirring for about 10 minutes. The pH is adjusted to 12 by addition of caustic lye solution (0.9 mL). The mass is extracted with ethyl acetate (2x20 mL). The organic layer is distilled completely under vacuum. The obtained residue is cooled to 0-5°C, ethyl acetate (2 mL) is added, and the mixture is stirred for about 5 minutes. The pH of the solution is adjusted to 2 by addition of hydrogen chloride in isopropanol (1 mL) and the mixture is stirred for solid formation. The solid is filtered and washed with ethyl acetate, and then suction dried for 45 minutes to afford 0.4 g of the title compound.

### EXAMPLE 3: PREPARATION OF PROPARGYLAMINE HYDROCHLORIDE.

Propargylamine (200 g) is charged into a round bottom flask containing ethyl acetate (1500 mL) and stirred for about 10 minutes. The solution is cooled to 0-5°C, pH is adjusted to 2-5 by addition of hydrogen chloride in isopropanol (18%, 1405 mL), and the mixture is stirred for solid formation. The solid is filtered and washed with ethyl acetate (400 mL), and then suction dried for about 30 minutes. The obtained solid is dried at 70°C for 6-7 hours to afford 300 g of the title compound.

### EXAMPLE 4: PREPARATION OF N-(2-PROPYNYL)-INDANYLAMINE.

1-Indanone (5 g), progargylamine hydrochloride (6.9 g), and methanol (50 mL) are charged into a round bottom flask and stirred for about 30 minutes at room temperature. To the solution, sodium acetate (3.1 g) and sodium cyanoborohydride (2.37 g) are added, and then the mixture is heated to reflux and maintained for completion of the reaction. The mass is distilled completely under vacuum to produce a residue. Water (500 mL) is added to the residue, the pH is adjusted to 1-2 by addition of aqueous HCl (5 mL), the mixture is washed with toluene (2×25 mL), and the aqueous layer is separated. The obtained aqueous layer pH is adjusted to 11-13 by addition of caustic lye (5 mL) and extracted with ethyl acetate (2×30 mL). The organic layer is separated, washed with water (2x30 mL), and is distilled completely under vaccum to afford 5.8 g of the title compound as a residue (89.6% yield).

### EXAMPLE 5: PREPARATION OF N-(2-PROPYNYL)-INDANYLAMINE.

1-Indanone (5 g) and progargylamine (4.16 g) are charged into a round-bottom flask containing methanol (50 mL) and methanolic hydrogen chloride (12.5 mL). Sodium cyanoborohydride (2.4 g) is added and the mixture is heated to reflux, then maintained for completion of the reaction. The mass is cooled to room temperature and the solvents are distilled completely under vacuum to obtain a residue. Water (50 mL) is added to the residue, pH is adjusted to 1-3 by adding aqueous HCl (5.6 mL), the mixture is extracted with toluene (2×25mL), and the aqueous layer is separated. The pH of the aqueous layer is adjusted to 11-13 by adding caustic lye (5.4 mL) and the layer is extracted with ethyl acetate (2×25 mL). The organic layer is washed with water (2×25 mL) and distilled completely under vacuum below 60°C, to afford 5.8 g of the title compound as a residue.

### EXAMPLE 6: PREPARATION OF N-(2-PROPYNYL)-INDANYLAMINE.

1-Indanone (2 g) and progargylamine 1.66 g are charged into a round-bottom flask containing methanol (20 mL) and hydrochloric acid (2 mL). Sodium cyanoborohydride (0.95 g) is added, then the mixture is heated to reflux and maintained for completion of the reaction. The mass is cooled to room temperature and the solvents are distilled completely under vacuum to obtain a residue. Water (20 mL) is added to the residue, pH is adjusted to 1-3 by adding aqueous HCl (1 mL), the mixture is extracted with toluene (2x5 mL), and the aqueous layer is separated. The pH of the aqueous layer is adjusted to 11-13 by adding caustic lye (1.9 mL) and it is extracted with ethyl acetate (2×5 mL). The organic layer is washed with water (2×5 mL) and distilled completely under vacuum to afford 2.2 g of the title compound as a residue.

### EXAMPLE 7: PREPARATION OF DIASTEREOMERIC SALT OF RASAGILINE.

Racemic rasagiline free base (2 g) is charged into a round-bottom flask containing 2-butanol (20 mL) and stirred for about 5 minutes. DPTTA (2.257g) is added to the solution, heated to 60°C, and maintained for about 45 minutes. The solution is cooled to room temperature and stirred for solid formation. The solid is filtered, washed with 2-butanol (4 mL), and suction dried for about 30 minutes to afford the title compound (1.1 g).
Purity by chiral HPLC: 82.02% R-isomer.

### EXAMPLE 8: PREPARATION OF DIASTEREOMERIC SALT OF RASAGILINE.

Racemic rasagiline free base (2 g) is charged into a round-bottom flask containing acetone (20 mL) and stirred for about 5 minutes. DPTTA (4.7 g) is added to the solution, heated to 60°C, and maintained for about 45 minutes. The solution is cooled to room temperature and stirred for solid formation. The solid is filtered, washed with acetone (4.7 mL), and suction dried for about 30 minutes to afford the title compound (1.9 g).
Purity by chiral HPLC: 88% R-isomer.

### EXAMPLE 9: PREPARATION OF DIASTEREOMERIC SALT OF RASAGILINE.

Racemic rasagiline free base (2 g) is charged into a round-bottom flask containing ethyl acetate (20 mL) and stirred for about 5 minutes. DPTTA (1.35 g) is added to the solution, heated to 40°C, and maintained for about 45 minutes. The solution is cooled to room temperature and stirred for solid formation. The solid is filtered, washed with ethyl acetate (4 mL), and suction dried for about 30 minutes to afford the title compound (1.3 g).
Purity by chiral HPLC: 83.69% R-isomer.

### EXAMPLE 10: PREPARATION OF DIASTEREOMERIC SALT OF RASAGILINE.

Racemic rasagiline free base (2 g) is charged into a round-bottom flask containing isopropanol (60 mL) and stirred for about 5 minutes. DPTTA (4.51 g) is added to the solution, heated to 40°C, and maintained for about 45 minutes. The solution is cooled to room temperature and stirred for solid formation. The solid is filtered, washed with isopropanol (10 mL), and suction dried for about 30 minutes to afford the title compound (1.3 g).
Purity by chiral HPLC: 93% R-isomer.

### EXAMPLE 11: PREPARATION OF DIASTEREOMERIC SALT OF RASAGILINE.

Racemic rasagiline free base (5 g) is charged into a round-bottom flask containing isopropanol (50 mL) and stirred for about 5 minutes. L-(+)-tartaric acid (1.315 g) is added to the solution, heated to 60°C, and maintained for about 45-60 minutes. The solution is cooled to 25-30°C and stirred for solid formation. The solid is filtered, washed with isopropanol (10 mL), and suction dried for about 30 minutes. The obtained solid is dried at 60°C to afford the title compound (3.3 g).
Purity by chiral HPLC: 94.80% R-isomer.

### EXAMPLE 12: PREPARATION OF DIASTEREOMERIC SALT OF RASAGILINE.

Racemic rasagiline free base (100 g) is charged into a round bottom flask containing a mixture of isopropyl alcohol (500 ml) and methanol (700 ml) and stirred for about 5 minutes. L-(+)-tartaric acid (26.3 g) is added to the solution, heated to 60°C, and maintained for about 45 minutes, followed by cooling to 25-30°C and stirring for solid formation. The solid is filtered, washed with isopropyl alcohol (100 mL) and suction dried for about 30 minutes. The solid is dried at 70°C to afford the title compound (49 g).
Chemical purity: 99.82%.
Purity by chiral HPLC: 99.25% R-isomer

### EXAMPLE 13: PREPARATION OF RASAGILINE MESYLATE.

A rasagiline tartrate salt (5 g) is charged into a round-bottom flask containing isopropanol (50 mL) and stirred for about 5 minutes. Methanesulfonic acid (2 g) is added to the solution, heated to reflux, and maintained for about 45 minutes. The solution is cooled to 10°C and stirred for solid formation. The solid is filtered, washed with isopropanol (10 mL), and dried under vaccum at 60°C to afford the title compound (2.8 g).
Purity by chiral HPLC: 99.27% R-isomer

### EXAMPLE 14: PREPARATION OF RASAGILINE MESYLATE.

A rasagiline tartrate salt (25 g) is charged into a round-bottom flask containing acetone (375 mL) and stirred for about 5 minutes. Methanesulfonic acid (11.22 g) is added to the solution, heated to reflux, and maintained for about 45 minutes. The solution is cooled to 25-30°C and stirred for solid formation. The solid is filtered, washed with acetone (50 mL), and dried under vaccum at 60°C to afford the title compound (19 g).
Purity by chiral HPLC: 100% R-isomer.

### EXAMPLE 15: CONVERSION OF (S)-N-(2-PROPYNYL)-INDANYLAMINE DIASTEROMERIC SALT INTO A RACEMIC MIXTURE OF RASAGILINE.

(S)-N-2-propynyl-1-indanylamine diastereomeric salt (100 g) and water (500 mL) are combined and stirred for about 15 minutes. The pH of the solution is adjusted to 10 by adding 40% aqueous sodium hydroxide solution and the mixture is stirred for about 20 minutes. Ethyl acetate (500 mL) is added to the solution and stirred for about 10 minutes. The organic layer is separated. The organic layer is distilled completely under vacuum to produce 48.8 g of a residue.

The residue (2 g) containing (S)-N-2-propynyl-1-indanamine, sodium hydroxide (0.5 g), and dimethylsulfoxide (10 mL) are placed into a round bottom flask and stirred for about 15 minutes. The mixture is heated to about 90°C and maintained for about 2 hours, followed by cooling to room temperature. Water (50 mL) is added and stirred for about 45 minutes. To the solution, dichloromethane (15 mL) is added and stirred for about 15 minutes. The organic layer is separated and distilled completely under vacuum below 45°C, to afford 1.3 g of racemic rasagiline.
Enantiomeric ratio (S:R) by chiral HPLC = 49.93:50.07.

### EXAMPLE 16: PREPARATION OF RASAGILINE TARTRATE.

In a round bottom flask, 1-indanone (100 g), methanol (1000 mL), propargylamine HCl (104 g), and sodium acetate trihydrate (62 g) are combined and stirred for 5-10 minutes. To the mixture, sodium cyanoborohydride (47.7 g) is added and the mass is stirred at 50-55°C for 3-7 hours, with thin layer chromatography used to confirm reaction completion. The solvent is distilled completely under vacuum below 60°C, followed by cooling to 25-35°C. Water (1000 mL) is added and pH is adjusted to 11-12.5 by adding caustic lye (45 mL), followed by extraction with ethyl acetate (2×500 mL). The organic layers are combined and washed with water (2×300 mL), followed by distillation under vacuum below 70°C. To the residue, ethyl acetate (100 mL) is added and distilled completely under vacuum below 70°C, producing a residue To the residue, methanol (890 mL), isopropyl alcohol (635 mL) and tartaric acid (33.4 g) are added and the mixture is stirred at about 25-35°C. The mixture is heated to 60-70°C with continuous stirring for 1-2 hours, followed by cooling to 25-35°C for 1-2 hours. The formed solid is filtered, washed with isopropyl alcohol (127 mL) and suction dried below 70°C for 6-8 hours, to give rasagiline tartrate in ∼77% yield.
Purity: rasagiline tartrate: 98.59%; S-propargylaminoindane tartrate: 1.41%.

### EXAMPLE 17: PREPARATION OF RASAGILINE TARTRATE.

Rasagiline tartrate (30 g) is charged into a round bottom flask containing methanol (450 mL). The mixture is heated to reflux for dissolution and stirred for 25-30 minutes, followed by addition of isopropyl alcohol (150 mL) at 55-65°C. The mixture is stirred at the same temperature for 25-30 minutes, followed by cooling to 25-35°C and stirring for 45-60 minutes. The solid obtained is filtered, washed with isopropyl alcohol (60 mL) and dried at 60-70°C to afford the title compound in 76.6% yield.
HPLC purity: 99.85%.

### EXAMPLE 18: PREPARATION OF RASAGILINE TARTRATE.

Rasagiline tartrate (10 g) is charged into a round bottom flask containing methanol (150 mL). The mixture is heated to reflux for dissolution and stirred for 45-60 minutes at reflux followed by cooling to 25-30°C. The mixture is stirred at this temperature for 45-60 minutes. The solid obtained is filtered, washed with methanol (10 mL) and dried below 60°C to afford the title compound.
HPLC purity: 99.80%.

### EXAMPLE 19: PREPARATION OF RASAGILINE TARTRATE.

Rasagiline free base (8.0 g), L-(+)-tartaric acid (2.10 g), methanol (56 mL) and isopropyl alcohol (40 mL) are placed in a flask and the mixture is stirred for 5-15 minutes. The mixture is heated to reflux and maintained for 60-90 minutes under constant stirring. The mixture is then cooled to 25-35°C and stirred for 60-90 minutes. The solid obtained is filtered under vacuum, washed with isopropyl alcohol (8 mL), and dried for 6-8 hours at 60-70°C to afford the title compound.
HPLC purity is 99.94%

### EXAMPLE 20: PREPARATION OF RASAGILINE MESYLATE.

Rasagiline tartrate (200 g), is charged into a round bottom flask containing water and stirred to produce a solution, and pH is adjusted to 11.98 with aqueous sodium hydroxide solution under constant stirring. Rasagiline free base is extracted from the aqueous solution using two lots of ethyl acetate (930 mL and 1000 mL). The combined organic layer is washed with two lots of water (1070 mL and 1090 mL), followed by distillation under vacuum below 70°C. To the residue, ethyl acetate (200 ml) is added and distilled under vacuum to obtain a residue, which is analyzed by chromatography (R-isomer: 98.46%; S-isomer: 1.54%; chemical purity: 99.28%). The residue is dissolved in isopropyl alcohol (1200 mL), followed by addition of methanesulfonic acid (93.7 g) over 15 minutes. The mixture is stirred and heated to 75°C under constant stirring and maintained at that temperature for 10 minutes. The mixture is allowed to cool to 25°C, and then precipitated solid is filtered and washed with isopropyl alcohol (200 mL). The filtered solid is suction dried, then dried at 70°C for about 8 hours to give rasagiline mesylate in 83% yield.
Chiral purity: R-Propargylaminoindane mesylate = 99.95%; S-Propargyl aminoindane mesylate = 0.05%.
Chemical purity: Rasagiline mesylate >99.9%; N-Allylaminoindane <0.05%.

### EXAMPLE 21: PREPARATION OF RASAGILINE MESYLATE.

Rasagiline tartrate (730 g) is charged into a reactor containing water (14.6 L) and stirred to dissolve, and pH is adjusted to 11-12 with aqueous sodium hydroxide solution under constant stirring. Rasagiline free base is extracted from the aqueous solution using two lots of ethyl acetate (2x5.11 L). The combined organic layer is washed with water (3.65 L), followed by distillation of the organic layer under vacuum below 70°C. To the residue, isopropyl alcohol (10.95 L) is added, followed by addition of methanesulfonic acid (340 g) over 15 minutes. The mixture is stirred until solid separates, then is maintained under reflux for dissolution and stirred for 60-90 minutes. The mixture is allowed to cool to 25-35°C and stirred for 60-90 minutes, and then solid is separated by centrifugation and washed with isopropyl alcohol (730 mL). The solid is spin dried, then dried under vacuum at 60-70°C for about 8 hours to give rasagiline mesylate in 42% yield.

### EXAMPLE 22: PREPARATION OF RASAGILINE MESYLATE

Rasagiline tartrate (120 g) is charged into a reactor containing acetone (1800 mL) and stirred for about 5 minutes. Methanesulfonic acid (48.1 g) is added to the mixture and dissolved, then the mixture is heated to reflux and maintained for about 45-60 minutes. The solution is cooled to 25-35°C and stirred for 30-45 minutes for solid formation. The solid is filtered, washed with acetone (240 mL), and dried under vaccum at 60°C to a constant weight. The dry solid is mixed with acetone (94 mL) in a flask, heated to reflux, and maintained for about 45-60 minutes. The mixture is cooled to room temperature and stirred for 45-60 minutes. The solid obtained is filtered, washed with acetone (189 mL), and dried at 55-60°C to a constant weight. The dry compound is combined with isopropyl alcohol (400 mL) in a flask and heated to reflux for dissolution, then maintained for 20-30 minutes. The mixture is cooled to 25-35°C for solid formation. Acetone (800 mL) is added to the mixture at 25-35°C and stirred for 45-60 minutes. The solid obtained is filtered under vacuum, washed with acetone (160 mL), and dried at 55-60°C to afford the title compound in 60.4% yield.
Purity: 99.91%.

### EXAMPLE 23: PREPARATION OF RASAGILINE MESYLATE.

Rasagiline tartrate (10 g) is charged into a reactor containing isopropyl alcohol (10 mL). Methanesulfonic acid (4.0 g) is added and dissolved, then the mixture is heated to reflux and maintained for about 60 minutes. The solution is cooled to 0-5°C and stirred for 30 minutes for solid formation. The solid is filtered, washed with isopropyl alcohol (10 mL), and dried at below 80°C to afford the title compound in 65% yield.
Purity: 99.90%.

### EXAMPLE 24: PREPARATION OF RASAGILINE MESYLATE.

Rasagiline mesylate (1.0 g) is charged into a flask containing methanol (4 mL). The solvent is distilled under vacuum below 20°C. A vacuum is applied after distillation for about 2-3 hours.

### EXAMPLE 25: PREPARATION OF RASAGILINE MESYLATE.

Rasagiline mesylate (1.0 g) is charged into a flask containing water (5 mL). The solution is cooled to -40 to -70°C using dry ice and methanol and then lyophilized under a vacuum of 185-196 mtorr for 8-10 hours, to afford title compound in 1.1 g yield.

### EXAMPLE 26: PREPARATION OF RASAGILINE MESYLATE.

Rasagiline tartrate (3 kg) is charged into a reactor containing water (60 L) and stirred to produce a solution, and pH is adjusted to 11-12.5 with aqueous sodium hydroxide solution under constant stirring at 25-35°C. Rasagiline free base is extracted from the aqueous solution with ethyl acetate (2×21 L). The combined organic layer is washed with water (2×21 L), followed by distillation of the organic layer under vacuum below 55°C. To the residue, isopropyl alcohol (6 L) is added followed by distillation under vacuum below 60°C. Isopropyl alcohol (20 L) is added to the residue, followed by addition of methanesulfonic acid (1.35 Kg) at 25-35°C under a nitrogen atmosphere over 15 minutes. The mixture is stirred at 65-75°C for dissolution and maintained for 10-30 minutes. The mixture is allowed to cool to 25-35°C and maintained for 45-60 minutes under a nitrogen atmosphere, and then solid is centrifuged and washed with isopropyl alcohol (6 L). The solid is spin dried, then dried under vacuum at 60-65°C for about 8 hours to give rasagiline mesylate in 57% yield.
HPLC purity: 99.5%.

### EXAMPLE 27: PREPARATION OF RASAGILINE MESYLATE HAVING D₉₀ BELOW 6 µm.

Rasagiline mesylate (10 g) is charged into a dry round-bottom flask containing isopropyl alcohol (100 mL), heated to 75°C, and stirred to produce a clear solution. The solution is filtered hot to remove particles and the filtrate is poured into chilled methyl t-butyl ether (500 mL) at 2°C, then stirred for solid formation for about 5-10 minutes. The solid is filtered and suction dried for about 5-20 minutes. The solid is dried under vacuum at 50-60°C for about 4 hours. The dry solid is sifted through a 40 mesh sieve to get uniform particle sizes.

The obtained fine compound (8.5 g) is micronised under 5.6 Kg/cm² pressure for about 15 minutes to produce a particle size distribution having D₉₀ = 5.517 µm, D₁₀ = 0.779 µm, and D₅₀ = 2.285 µm
Chiral HPLC: S-isomer not detected.

### EXAMPLE 28: PREPARATION OF RASAGILINE MESYLATE HAVING D₉₀ BELOW 6 µm.

Rasagiline mesylate (5 g) is charged into a dry round bottom flask containing isopropyl alcohol (50 mL), heated to 75°C and stirred to produce a clear solution. The solution is filtered hot to remove particles and subsequently poured into chilled methyl t-butyl ether (240 mL) at 2°C and stirred for solid formation for about 5-10 minutes. The solid is filtered and suction dried for about 5-20 minutes. The solid is dried under vacuum at 50-60°C for about 4 hours. The dry solid (3.4 g) is sifted through an 80 mesh sieve to get uniform particle sizes.

The obtained fine compound (2.5 g) is micronised under 5.6 Kg/cm² pressure for about 15 minutes to produce a particle size distribution having D₉₀ = 3.219 µm, D₁₀ = 0.663 µm, D₅₀ = 1.592 µm, and D[4,3] = 1.792 µm.

### EXAMPLE 29: PREPARATION OF RESAGILINE MESYLATE WITH REDUCED PARTICLE SIZES BY RECRYSTALLIZATION.

Rasagiline tartrate (120 g) is charged into a round bottom flask containing acetone (1800 mL) and stirred, followed by addition of methanesulfonic acid (48.1 g). The mixture is heated to reflux and maintained for 45-60 minutes, followed by cooling to 25-35°C with constant stirring for 30-45 minutes. The formed solid is filtered, washed with acetone (240 mL), dried by suction, and dried at 55-60°C to a constant weight. The dry solid is placed into another flask containing acetone (940 mL) and the mixture is heated to reflux and maintained for 45-60 minutes, followed by cooling to room temperature and continuous stirring for another 45-60 minutes. The formed solid is filtered under vacuum and washed with acetone (189 mL), followed by suction drying and drying at 55-60°C to a constant weight. The dry compound is charged into a flask containing isopropyl alcohol (400 mL) and refluxed for 20-30 minutes. The mixture is allowed to cool to 25-35°C, followed by addition of acetone (800 mL) at 25-35°C. The mixture is stirred for 45-60 minutes and formed solid is filtered under vacuum, washed with acetone (160 mL), suction dried, and dried at 55-60°C to a constant weight, giving the product in 60.4% yield.
Chiral purity: 100% R-isomer.

### EXAMPLE 30: PREPARATION OF RESAGILINE MESYLATE WITH REDUCED PARTICLE SIZES BY SLURRYING.

Rasagiline mesylate (15 g) obtained by a procedure according to Example 21 is slurried in methyl t-butyl ether (600 mL) at room temperature for 2 hours. The solid is filtered, washed with methyl t-butyl ether (60 ml), and suction dried for 15 minutes. The solid is dried for 8 hours at 70°C. The dried solid is micronised under a pressure of 4-5 Kg/cm² to produce a particle size distribution having D₉₀ = 4.981 µm, D₁₀ = 0.761 µm, D₅₀ = 2.346 µm, and D[4,3] = 2.650 µm.

### EXAMPLE 31: PREPARATION OF RASAGILINE MESYLATE.

Rasagiline tartrate (1 kg) is charged into a reactor containing water (20 L) and stirred at 25-35°C to produce a solution, and pH is adjusted to 11-12.5 with 40% aqueous sodium hydroxide solution (0.62 L) under constant stirring at 25-35°C. Rasagiline free base is extracted from the aqueous solution with ethyl acetate (2×7 L). The combined organic layer is washed with water (5 L), followed by distillation of the organic layer under vacuum below 70°C. To the residue, isopropyl alcohol (15 L) is added followed by addition of methanesulfonic acid (0.47 Kg) at 25-35°C. The mixture is stirred at reflux for dissolution and maintained for 60-90 minutes. The mixture is allowed to cool to 25-35°C and maintained for 60-90 minutes under a nitrogen atmosphere, and then formed solid is centrifuged and washed with isopropyl alcohol (1 L). The solid is spin dried, then dried under vacuum at 60-65°C for about 8 hours to give rasagiline mesylate in 50.6 % yield. The dried solid is micronized under a pressure of 4-5 Kg/cm² to produce a particle size distribution having D₉₀ = 608.570 µm, D₁₀ = 2.404 µm, and D₅₀ = 146.957 µm.

### EXAMPLE 32: PREPARATION OF RESAGILINE MESYLATE WITH REDUCED PARTICLE SIZES BY SLURRYING.

Rasagiline mesylate (5 g) obtained by a procedure according to Example 31 is slurried in methyl t-butyl ether (200 mL) at room temperature for 2 hours. The solid is filtered, washed with methyl t-butyl ether (20 mL), and suction dried for 15 minutes. The solid is dried for 8 hours at 70°C. The dried solid is micronized under a pressure of 4-5 Kg/cm² to produce a particle size distribution having D₉₀ = 6.540 µm, D₁₀ = 0.991 µm, D₅₀ = 3.093 µm, and D[4,3] = 3.496 µm.

### EXAMPLE 33: AGGLOMERATION OF MICRONIZED RASAGILINE MESYLATE DURING STORAGE.

Rasgiline mesylate (6 g) prepared according to Example 28 is divided into three parts for storage under ambient conditions (temperature = 25±2°C; relative humidity = 65±5%): unpackaged (P-I); in closed packages with a nitrogen atmosphere (P-II); and in closed packages with an air atmosphere (P-III). The samples are analyzed for particle size distribution on the 5^{th} and 18^{th} days. The particle size distribution of the sample packaged with a nitrogen atmosphere is substantially unchanged during the experiment, as shown below.

| **Sample** | | **µm** | | |
|---|---|---|---|---|
| | | **D₁₀** | **D₅₀** | **D₉₀** |
| P-I | 5^{th} Day | 1.319 | 4.216 | 151.379 |
| | 18^{th} Day | ----- | ----- | ----- |
| P-II | 5^{th} Day | 0.892 | 2.859 | 5.524 |
| | 18^{th} Day | 1.065 | 3.227 | 6.282 |
| P-III | 5^{th} Day | 1.182 | 3.566 | 120.731 |
| | 18^{th} Day | 1.365 | 4.171 | 144.617 |

There have been disclosed hereinbefore compounds, rasagiline, rasagiline mesylate particles, crystalline forms, processes and methods defined by the following numbered paragraphs:
1. A process for preparing rasagiline or a pharmaceutically acceptable salt thereof, comprising:
   (a) reacting 1 -indanone of Formula II with propargylamine or a salt thereof, in the presence of a solvent, to afford N-(2-propynyl)-indanylimine of Formula III or a salt thereof, which is optionally isolated; and 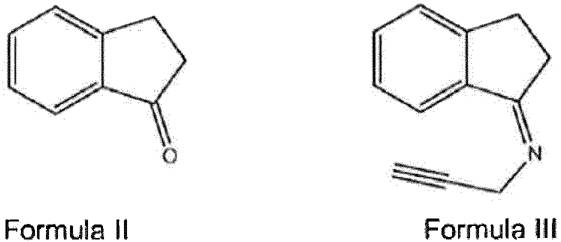
   (b) reducing N-(2-propynyl)-indanylimine of Formula III or a salt thereof with a reducing agent, to afford a compound of Formula IV. 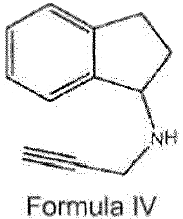
2. The process according to paragraph 1 , wherein a solvent comprises an alcohol, an aromatic hydrocarbon, a halogenated hydrocarbon, or an ether.
3. The process according to paragraph 1 , wherein a solvent comprises methanol.
4. The process according to paragraph 1 , wherein a reducing agent comprises Raney nickel, palladium on carbon, platinum dioxide, lithium aluminium hydride, sodium borohydhde, sodium cyanoborohydhde, or sodium bis(2-methoxyethoxy)aluminum hydride.
5. The process of paragraph 1 , further comprising:
   (i) reacting a compound of Formula IV with a chiral resolving agent to afford a diasteromeric salt;
   (ii) optionally, obtaining the free base of an enantiomer from a diastereomeric salt; and (iii) reacting the free base obtained in step (ii), or a diastereomeric salt obtained in step (i), with an acid in a solvent to afford an acid addition salt of rasagiline.
6. The process of paragraph 5, wherein a chiral resolving agent comprises L-(+)-tartaric acid, (-)-di-p-toluoyltartaric acid, a mandelic acid, or a camphorsulphonic acid.
7. The process of paragraph 5, wherein a solvent in (iii) comprises an alcohol, a ketone, an aromatic hydrocarbon, an ester, a halogenated hydrocarbon, an ether, a nitrile, or any mixture thereof.
8. The process of paragraph 5, wherein an acid comprises methanesulfonic acid.
9. Rasagiline mesylate particles having a particle size distribution with D₉₀ less than or equal to about 6 µm.
10. A process for preparing rasagiline mesylate particles having a particle size distribution with D₉₀ less than or equal to about 6 µm, comprising: a) providing a solution of rasagiline mesylate in isopropyl alcohol; b) combining the rasagiline mesylate solution of a) with an anti-solvent; c) recovering solid rasagiline mesylate from b); and d) reducing particle sizes of rasagiline mesylate to obtain a particle size distribution with D₉₀ less than about 6 µm.
11. The process of paragraph 10, wherein an anti-solvent comprises an ether.
12. The process of paragraph 10, wherein an anti-solvent comprises methyl t- butyl ether.
13. A process for preparing rasagiline mesylate particles having D₉₀ less than or equal to about 6 µm, comprising: a) slurrying rasagiline mesylate in an ether; b) recovering solid rasagiline mesylate from a), and c) reducing particle sizes of rasagiline mesylate to obtain D₉₀ less than about 6 µm.
14. The process of paragraph 13, wherein an ether comprises methyl t-butyl ether.
15. Crystalline rasagiline mesylate prepared by a process of paragraph1 , containing less than about 0.05 percent by weight of 3-propynylamino-1 -indanone or a salt thereof.

## Claims

1. Rasagiline or its salt wherein the amount of 3-propynylamino-1-indanone is less than 0.1 % by weight.

2. Rasagiline or its salt wherein the amount of 3-propynylamino-1-indanone is less than 0.05% by weight.

3. The compound of claim 1 wherein salt of rasagiline is a mesylate salt.

4. The compound of claim 1 wherein salt of rasagiline is a tartrate salt.

5. Rasagiline mesylate particles having a particle size distribution with D₉₀ less than or equal to about 6 µm.

6. A process for preparing rasagiline mesylate particles having a particle size distribution with D₉₀ less than or equal to about 6 µm, comprising:
a) providing a solution of rasagiline mesylate in isopropyl alcohol;
b) combining the rasagiline mesylate solution of a) with an anti-solvent;
c) recovering solid rasagiline mesylate from b); and
d) reducing particle sizes of rasagiline mesylate to obtain a particle size distribution with D₉₀ less than about 6 µm.

7. The process of claim 6, wherein an anti-solvent in step b) comprises an ether.

8. The process of claim 6, wherein an anti-solvent in step b) comprises methyl t-butyl ether.

9. A process for preparing rasagiline mesylate particles having D₉₀ less than or equal to about 6 µm, comprising:
a) slurrying rasagiline mesylate in an ether;
b) recovering solid rasagiline mesylate from a), and
c) reducing particle sizes of rasagiline mesylate to obtain D₉₀ less than about 6 µm.

10. The process of claim 9, wherein an ether comprises methyl t-butyl ether.
